# EUROPEAN PATENT APPLICATION

(11) **EP 2 292 730 A1**
(43) Date of publication of application: **09.03.2011**
(21) Application number: 10290470.3
(22) Date of filing: 02.09.2010
(51) Int. Cl.: C12M 1/26

(54) **Sampling method and device for the analysis of a surface**

(30) Priority: 03.09.2009 FR 0956019
(71) Applicant: Millipore Corporation, Billerica, MA 01821 (US)
(72) Inventor: Stephan, Nolwenn, 67400 Illkirch (FR)
(74) Representative: Lepelletier-Beaufond, François

(57) **Abstract**

The method comprises the step of applying a substance (7) to the surface to analyze (45), which has an affinity with microorganisms and which is adapted to be cultured to identify and count the microorganisms which were transferred during said applying step; which substance is a microporous membrane (7) for microbiological analysis, of hydrophilic synthetic material, made humid at least at the end of the applying step with an aqueous liquid such as water or saline solution.

The device comprises a substance (7) having an affinity with microorganisms which may be present on a surface (45), means for humidifying (6) said substance (7) with an aqueous liquid such as water or saline solution and means (12) for applying said substance (7) to said surface (45), said substance being a microporous membrane (7) for microbiological analysis, of hydrophilic synthetic material.

## Description

The present invention concerns sampling for the microbiological analysis of a surface.

A known method of sampling consists of using a contact or Petri dish containing gel growth medium. The slightly convex surface of that gel growth medium is applied against the surface to analyze for a predetermined period of time. The microorganisms that may be present on the surface to which the gel growth medium is applied are transferred onto the gel growth medium due to its sticky character.

This gel growth medium is then cultured at a temperature adapted to the growth of the microorganisms until they become identifiable and able to be counted, which may take several days.

Traces of the gel growth medium remain on the sampled surface after its removal, the nutritive character of the traces being liable to promote the proliferation of the microorganisms. For these reasons, as soon as the sampling has been carried out, that surface is cleaned with a sterile humid pad or with a disinfectant.

The invention aims to improve the sampling conditions on a surface which may comprise microorganisms.

To that end, according to a first aspect, it provides a sampling method for the microbiological analysis of a surface, comprising the step of applying a substance to said surface which has an affinity with microorganisms that may be present on said surface, which substance is adapted to be treated after said applying step to reveal possible said microorganisms transferred from said surface to said substance in said applying step; **characterized in that** said substance is a microporous membrane for microbiological analysis, of hydrophilic synthetic material, which is made humid at least at the end of said applying step with an aqueous liquid such as water or saline solution.

The method according to the invention makes it possible to perform the sampling directly with a conventional microporous membrane, of hydrophilic synthetic material.

Surprisingly, it turns out that the simple fact of making such a membrane humid at least at the end of the applying step, including simply with water, enables that membrane to have a sufficient affinity with the microorganisms which may be present on the surface to analyze, such that they are transferred to the membrane.

This affinity is probably due to the capillary forces acting between the membrane and the water molecules situated on the surface to analyze.

Thus, the use of a sticky substance (such as gel growth medium) is in no way necessary to sample the microorganisms.

Furthermore, the fact of using as sampling substance a synthetic microporous membrane for microbiological analysis enables all the techniques currently known for the analysis of liquids to be employed.

Thus, after the sampling of the microorganisms, the membrane may be analyzed in conventional manner by culturing on a nutritive gel growth medium or may be analyzed by fast microbiology.

According to features preferred for reasons of simplicity, convenience and economy, with regard to both manufacture and use:
- said membrane is made humid by imbibing the aqueous liquid;
- said membrane is of synthetic polymer selected from the group formed by polyvinylidene fluoride (PVDF), cellulose ester (CE) and polyethersulfone (PES);
- said membrane has a pore diameter between 0.1 and 1µm;
- said applying step comprises the step of placing a first face of said membrane in contact with said surface and the step of pressing an absorbent substrate imbibed with the aqueous liquid against a second face of said membrane that is the reverse of said first face;
- said absorbent substrate is elastically deformable;
- said absorbent substrate is a sponge;
- said pressing step is carried out for a predetermined time at least equal to 5 seconds;
- said predetermined time is between 10 and 20 seconds;
- said pressing step is performed manually;
- said applying step comprises, after said pressing step, the step of releasing the pressure exerted on said substrate to cause lifting of the aqueous liquid and of possible said microorganisms sampled from said surface;
- said sampling method comprises, prior to said applying step, the step of humidifying said surface with the aqueous liquid; and/or
- said step of humidifying said surface is carried out by spraying with the aqueous liquid.

According to a second aspect, the invention provides a sampling device for the microbiological analysis of a surface, comprising a substance having an affinity with microorganisms that may be present on said surface, which substance is adapted to be treated after said sampling to reveal possible said microorganisms transferred from said surface to said substance in said sampling, and comprising means for applying said substance to said surface; **characterized in that** said substance is a microporous membrane for microbiological analysis, of hydrophilic synthetic material, and in that said device comprises means for humidifying said microporous membrane with an aqueous liquid such as water or saline solution.

According to features preferred for reasons of simplicity, convenience and economy, with regard to both manufacture and use:
- said applying means comprise a plunger adapted to slide within a belt;
- said belt comprises a rigid cylindrical lateral wall projecting from a foot, said cylindrical lateral wall and said plunger being linked by an annular channel member with a U-shaped cross-section adapted to deform during the sliding of said plunger;
- said foot comprises a circular edge adapted to rest against said surface during said sampling to form a barrier to liquids;
- said belt, said plunger and said channel member are of plastics material formed as a one-piece molding;
- said humidifying means comprise a sponge imbibed with the aqueous liquid in contact with said membrane, said sponge being disposed in the cavity partially delimited by said belt;
- said sampling device has a packaging configuration in which said sponge is enclosed in a fluid-tight envelope to avoid the evaporation of the aqueous liquid contained in said sponge, and a configuration for use in which said sponge is in communication with the external air;
- said envelope comprises a sheet of aluminum disposed between said plunger and said sponge, and a plastic film disposed facing a face of said membrane that is the reverse of the face facing said sponge;
- said plunger comprises a plurality of teeth extending towards said aluminum sheet, said teeth being adapted to pierce said aluminum sheet during the sliding of said plunger;
- said plunger comprises a rigid disc, said teeth extending transversely to the plane of said disc and being regularly distributed over its periphery;
- said plunger comprises rigidifying means; and/or
- said rigidifying means comprise four flat ribs extending in a star-shaped arrangement.

The disclosure of the invention will now be continued with the detailed description of an embodiment, given below by way of illustrative but non-limiting example, with reference to the accompanying drawings, in which:
- Figure 1 is a partially cut away perspective view of the device according to the present invention;
- Figure 2 is a corresponding section view in elevation of the sampling unit alone;
- Figure 3 is a partially cut away perspective view from another angle of the body formed from molded plastics material which the sampling unit comprises; and
- Figure 4 is a partially cut away perspective view, from an angle similar to that of Figure 1, of the sampling unit in pushed-in position and applied to a surface which may comprise microorganisms.

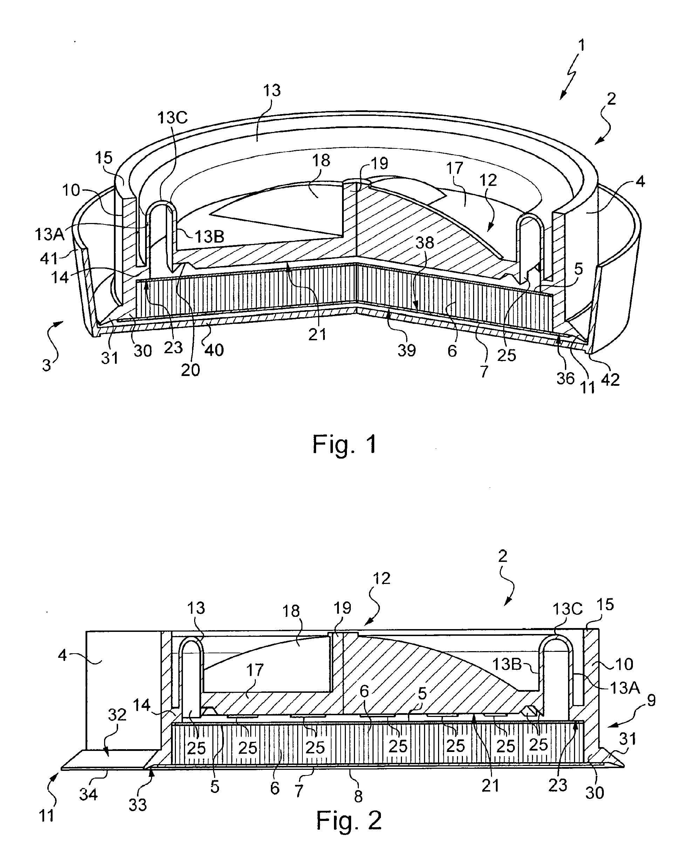

The device 1 for sampling microorganisms illustrated in Figure 1 in its packaging configuration, comprises a sampling unit 2 and a dish 3.

The sampling unit 2 (Figure 2) comprises a body 4 of molded plastics material, a sheet 5, a sponge 6, a hydrophilic microporous membrane 7 and a film 8.

The body 4 comprises a belt 9 formed by a rigid cylindrical lateral wall 10 projecting from a foot 11, a plunger 12 and an annular channel member 13 with a U-shaped cross-section linking the wall 10 to the plunger 12.

The annular channel member 13 and the lateral wall 10 are concentric, the free edge of the outer wall 13A of the annular channel member 13 being connected to the lateral wall 10 via a rib 14 projecting from the inside face of that wall 10 over the whole of its circumference.

The channel member 13 extends towards the free edge 15 of the wall 10, its intermediate wall 13C, here semicircular, being situated slightly recessed relative to that free edge 15.

The plunger 12 comprises a disc 17 surmounted by four flat ribs 18 extending in a star arrangement from a central stud 19, the stud 19 and the ribs 18 extending transversely to the plane of the disc 17.

An annular recess 20 (Figure 1) is formed at the periphery of the disc 17, on the face 21 thereof that is the reverse of the face from which project the ribs 18. This recess 20 extends to the free edge of the disc 17 which is connected at a right angle to the free edge of the inside wall 13B of the channel member 13, such that the disc 17 extends transversely to the lateral wall 10.

As the inside wall 13B of the channel member 13 is slightly shorter than its outside wall 13A, the face 21 of the disc 17 is slightly raised relative to the face 23 of the rib 14 (the one that is the reverse of the face from which project the ribs 18).

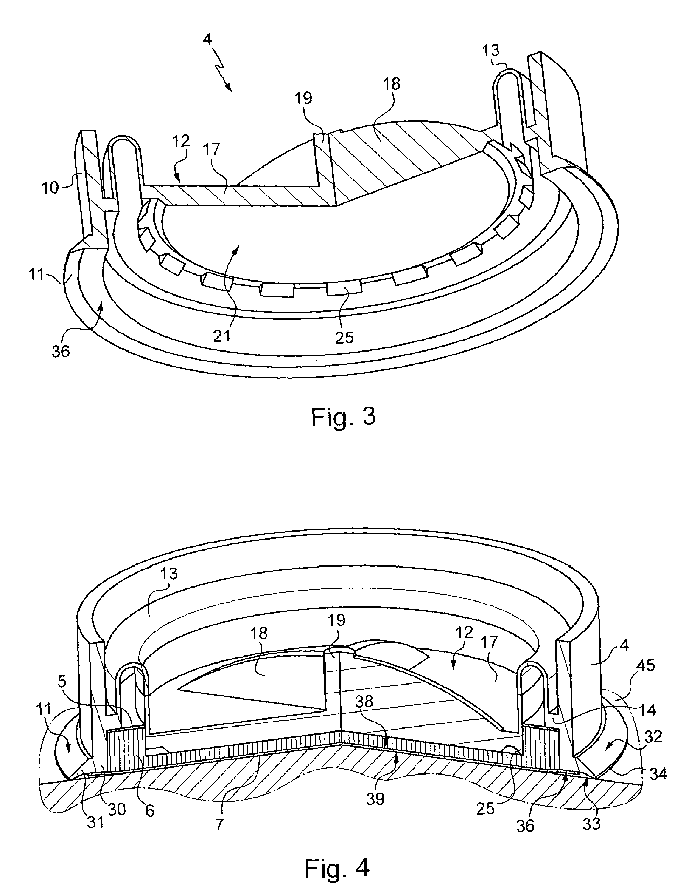

At the free edge of the disc 17, a plurality of teeth 25 extend transversely to the plane of the disc 17, in the opposite direction to the inside wall 13B of the channel member 13. These teeth 25 are regularly distributed over the whole of the periphery of the disc 17 and each have a beveled face directed towards the centre of that disc 17 (Figure 3), their respective ends being disposed in the same plane intermediate between that formed by the face 21 and that formed by the face 23 (Figure 2).

The foot 11 comprises an annular heel 30, here having a rectangular section, on which rests the lateral wall 10; as well as a peripheral rim 31 extending outwardly of the body 4 while slanting slightly away from the free edge 15.

The two frusto-conical faces 32 and 33 (Figure 2) of the peripheral rim 31 meet to form a circular edge 34 provided to rest on a surface 45 (Figure 4) which may include microorganisms, during the sampling operations.

The sheet 5 has a circular outline of which the diameter matches that of the inside face of the lateral wall 10. It is peripherally fixed by a welded (or bonded) joint to the face 23 of the rib 14, such that a slight gap separates the sheet 5 and the teeth 25 of the disc 17 facing the sheet 5 (Figure 2). This sheet 5 is here of aluminum in order to facilitate its piercing by the teeth 25 on implementation of the sampling method which will be disclosed in detail below.

The sponge 6 which is pre-imbibed with sterile water has a circular outline of the same diameter as that of the aluminum sheet 5. The sponge 6 is disposed in the cavity delimited by the sheet 5 and the inside faces of the lateral wall 10 and of the heel 30; it is of a predetermined thickness such that the end thereof remote from the sheet 5 is located flush with the face 36 of the heel 30 remote from the wall 10.

The hydrophilic membrane 7 is formed from synthetic polymer, preferably from polyvinylidene fluoride (PVDF), from cellulose ester (CE) or from polyethersulfone (PES) and the diameter of its pores is between 0.1 and 1µm.

The membrane 7 also has a circular outline but of which the diameter is equal to the outside diameter of the annular heel 30 (and therefore greater than that of the sheet 5 and of the sponge 6).

A first face 38 (Figure 1) of that membrane 7 is peripherally fixed by a welded (or bonded) joint to the face 36 of that annular heel 30, such that the sponge 6 is sandwiched between the membrane 7 and the aluminum sheet 5.

The film 8 has a circular outline of which the diameter is equal to that of the circular edge 34 to which it is fixed by a welded or bonded joint. This film 8 thus faces the second face 39 of the membrane 7 that is the reverse of the face facing the sponge 6.

It will be noted that in the packaging configuration of the device 1, the sheet 5 and the film 8 that are fixed to the body 4 by welded joints form a fluid-tight envelope isolating the sponge 6 and the membrane 7 from the outside environment, such that the purified water contained therein cannot evaporate to the atmosphere.

The film 8 is here of flexible plastic in order to limit the risk of accidental piercing before implementation of the sampling method.

The dish 3 comprises a circular bottom wall 40 of which the diameter is very slightly greater than that of the circular edge 34 of the body 4, and a frusto-conical lateral wall 41 that flares out from the bottom wall 40.

This frusto-conical wall 41 extends slightly beyond the bottom wall 40, so as to form a circular rib 42.

As can be seen in Figure 1, the sampling unit 2 is disposed partially within the dish 3 by the side comprising the membrane 7.

This disposition makes it possible to protect this membrane 7, which is fragile, against external shocks until an operator decides to perform a sampling operation.

In order to ensure provisional holding between the unit 2 and the dish 3, the latter is provided with retaining means, here ridges (not visible in the drawings).

A description will now be given of the different steps of the method implemented using the sampling device 1 which has just been described.

In a first phase, the operator takes the sampling unit 2 out of the protective dish 3, then removes the plastic film 8 from the body 4 by pulling it off for example using a small projecting tab provided for that purpose. The device 1 is then in configuration for use in which the sponge 6 and the membrane 7 are in communication with the external air.

The operator next puts the foot 11 of the body 4 onto the surface 45 where the microorganisms to collect may be located, then presses on the disc 17 of the plunger 12 with his fingers, towards the surface 45.

The plunger 12 slides within the belt 9, this movement being accompanied by the elastic deformation of the channel member 13.

The teeth 25 of the plunger 12 oriented towards the aluminum sheet 5 very rapidly enter into contact with it and pierce it at the periphery.

The continuation of the movement causes the tearing of the aluminum sheet 5 and the compression of the sponge 6 (Figure 4) against the membrane 7 which deforms elastically and of which the face 39 comes to rest against the surface 45. The presence of the ribs 18 confers a certain degree of rigidity to the disc 17 enabling the application of a uniform pressure on the sponge 6.

The sponge 6 then partially disgorges the water that it initially contained, this water being evacuated towards the surface 45 by passing through the pores of the hydrophilic membrane 7.

The circular edge 34 of the foot 11 which rests against the surface 45, forms a barrier to liquids, such that the water which evacuates from the sponge 6 remains contained on the inside of the edge 34.

The sampling unit 2 is then in the pushed-in position illustrated in Figure 4.

The force exerted by the operator on the plunger 12 is maintained for at least 5 seconds and preferably between 10 and 20 seconds.

During this period, possible microorganisms are bathed by the water molecules present on the surface 45, the circular edge 34 enabling them to be contained in the sampling zone.

The operator next releases the plunger 12 which progressively returns to its original position (Figure 2), as does the sponge 6. By resuming its initial volume, this sponge exerts a suction force which acts in addition to the capillarity to cause lifting of the water molecules and of the sampled microorganisms.

These organisms which have diameters greater than the pores of the membrane 7, come to be deposited on the surface of that humid membrane 7 whereas the water molecules are reabsorbed for the most part by the sponge 6.

In order to improve the retrieval ratio of possible microorganisms on the membrane 7, the steps of pressing then releasing the plunger 12 may be repeated several times.

Once the membrane 7 has been separated from the sampling unit 2, it may be directly cultured to reveal and count possible microorganisms which may have transferred from the surface 45 to that membrane 7.

For this purpose, the face 38 of the membrane 7 (that is the reverse of the face 39 on which the microorganisms are located) is placed directly onto a nutritive gel growth medium and then placed in incubation under conditions of temperature that are adapted to the development of those microorganisms.

The sampling may also be the subject of an analysis by fast microbiology, for example by detection of the ATP of the microorganisms present on the surface of the membrane 7.

In a variant not shown of the sampling method, the operator starts by depositing a membrane similar to the membrane 7, but dry, on the surface 45 that may comprise microorganisms.

He then covers the membrane with a humid sponge (or humid pad), then (directly with his fingers or via a plunger) presses on that sponge (or that pad) for at least 5 seconds and preferably between 10 and 20 seconds.

It is to be noted that this step may also be performed via a motorized applicator provided with a drive system which maintains a uniform pressure on the sponge or pad for a predetermined time.

In another variant of the sampling method according to the invention, the operator starts by humidifying the surface 45, by spraying for example sterile water, then places a similar membrane to membrane 7, but dry, on the surface 45 that may comprise microorganisms.

He then covers the membrane with a dry sponge (or dry pad), then, in the same way as disclosed above, presses on that sponge (or on that pad) for at least 5 seconds and preferably between 10 and 20 seconds.

According to still another variant of the method according to the invention, not shown, a humid membrane, similar to the membrane 7, is simply deposited on the surface 45 then removed after a predetermined period greater than one minute.

The membrane may also be placed, dry, on the surface 45 that is humidified in advance; and/or an aqueous liquid other than water may be used to humidify the membrane, for example a saline solution.

Numerous other variants are possible according to circumstances, and in this connection it is to be noted that that the invention is not limited to the example embodiments described and shown.

## Claims

1. A sampling method for the microbiological analysis of a surface (45), comprising the step of applying a substance to said surface (45) which has an affinity with microorganisms that may be present on said surface (45), which substance is adapted to be treated after said applying step to reveal possible said microorganisms transferred from said surface (45) to said substance in said applying step; **characterized in that** said substance is a microporous membrane (7) for microbiological analysis, of hydrophilic synthetic material, which is made humid at least at the end of said applying step with an aqueous liquid such as water or saline solution.

2. A sampling method according to claim 1, **characterized in that** said membrane (7) is made humid by imbibing the aqueous liquid.

3. A sampling method according to one of claims 1 or 2, **characterized in that** said membrane (7) is of synthetic polymer selected from the group formed by polyvinylidene fluoride (PVDF), cellulose ester (CE) and polyethersulfone (PES).

4. A sampling method according to one of claims 1 or 2, **characterized in that** said membrane (7) has a pore diameter between 0.1 and 1µm.

5. A sampling method according to one of claims 1 to 5, **characterized in that** said applying step comprises the step of placing a first face (39) of said membrane (7) in contact with said surface (45) and the step of pressing an absorbent substrate (6) imbibed with the aqueous liquid against a second face (38) of said membrane (7) that is the reverse of said first face.

6. A sampling method according to claim 5, **characterized in that** said absorbent substrate (6) is elastically deformable.

7. A sampling method according to claim 6, **characterized in that** said absorbent substrate is a sponge (6).

8. A sampling method according to one of claims 5 to 7, **characterized in that** said pressing step is carried out for a predetermined time at least equal to 5 seconds.

9. A sampling method according to claim 8, **characterized in that** said predetermined time is between 10 and 20 seconds.

10. A sampling method according to one of claims 5 to 9, **characterized in that** said pressing step is performed manually.

11. A sampling method according to one of claims 5 to 10, **characterized in that** said applying step comprises, after said pressing step, the step of releasing the pressure exerted on said substrate (6) to cause lifting of the aqueous liquid and of possible said microorganisms sampled from said surface (45).

12. A sampling method according to one of claims 1 to 4, **characterized in that** it comprises, prior to said applying step, the step of humidifying said surface (45) with the aqueous liquid.

13. A sampling method according to claim 12, **characterized in that** said step of humidifying said surface (45) is carried out by spraying with the aqueous liquid.

14. A sampling device for the microbiological analysis of a surface (45), comprising a substance having an affinity with microorganisms that may be present on said surface (45), which substance is adapted to be treated after said sampling to reveal possible said microorganisms transferred from said surface (45) to said substance in said sampling, and comprising means for applying (12) said substance to said surface (45) **characterized in that** said substance is a microporous membrane for microbiological analysis, of hydrophilic synthetic material, and **in that** said device comprises means for humidifying (6) said microporous membrane(7) with an aqueous liquid such as water or saline solution.

15. A sampling device according to claim 14, **characterized in that** said applying means comprise a plunger (12) adapted to slide within a belt (9).

16. A sampling device according to claim 15, **characterized in that** said belt (9) comprises a rigid cylindrical lateral wall (10) projecting from a foot (11), said cylindrical lateral wall (10) and said plunger (12) being linked by an annular channel member (13) with a U-shaped cross-section adapted to deform during the sliding of said plunger (12).

17. A sampling device according to claim 16, **characterized in that** said foot (11) comprises a circular edge (34) adapted to rest against said surface (45) during said sampling to form a barrier to liquids.

18. A sampling device according to one of claims 16 or 17, **characterized in that** said belt (9), said plunger (12) and said channel member (13) are of plastics material formed as a one-piece molding.

19. A sampling device according to one of claims 15 to 18, **characterized in that** said humidifying means comprise a sponge (6) imbibed with the aqueous liquid in contact with said membrane (7), said sponge (6) being disposed in the cavity partially delimited by said belt (9).

20. A sampling device according to claim 19, **characterized in that** it has a packaging configuration in which said sponge (6) is enclosed in a fluid-tight envelope (5, 8) to avoid the evaporation of the aqueous liquid contained in said sponge (6), and a configuration for use in which said sponge (6) is in communication with the external air.

21. A sampling device according to claim 20, **characterized in that** said envelope comprises a sheet of aluminum (5) disposed between said plunger (12) and said sponge (6), and a plastic film (8) disposed facing a face (39) of said membrane (7) that is the reverse of the face facing said sponge (6).

22. A sampling device according to claim 21, **characterized in that** said plunger (12) comprises a plurality of teeth (25) extending towards said aluminum sheet (5), said teeth (25) being adapted to pierce said aluminum sheet (5) during the sliding of said plunger (12).

23. A sampling device according to claim 22, **characterized in that** said plunger (12) comprises a rigid disc (17), said teeth (25) extending transversely to the plane of said disc (17) and being regularly distributed over its periphery.

24. A sampling device according to one of claims 15 to 23, **characterized in that** said plunger (12) comprises rigidifying means (18).

25. A sampling device according to claim 24, **characterized in that** said rigidifying means comprise four flat ribs (18) extending in a star-shaped arrangement.
